# EUROPEAN PATENT SPECIFICATION

(11) **EP 4 108 783 B1**
(45) Date of publication and mention of the grant of the patent: **21.02.2024**
(21) Application number: 21181309.2
(22) Date of filing: 24.06.2021
(51) Int. Cl.: C12Q 1/6841

(54) **SPATIAL SEQUENCING WITH MICTAG**
RÄUMLICHE SEQUENZIERUNG MIT MICTAG
SÉQUENÇAGE SPATIAL AVEC MICTAG

(43) Date of publication of application: 28.12.2022
(73) Proprietor: Miltenyi Biotec B.V. & Co. KG, 51429 Bergisch Gladbach (DE)
(72) Inventor: SPIECKER, Heinrich, 33617 Bielefeld (DE); BOSIO, Andreas, 51429 Bergisch Gladbach (DE); ROTHMANN, Thomas, 51429 Bergisch Gladbach (DE); JUNGPERTZ, Sandra, 51429 Bergisch Gladbach (DE)
(74) Representative: Kisters, Michael Marcus

(56) References cited:
- WO-A1-2011/127099
- WO-A1-2019/038372
- MERRITT CHRISTOPHER R ET AL: "Multiplex digital spatial profiling of proteins and RNA in fixed tissue", NATURE BIOTECHNOLOGY, NATURE PUBLISHING GROUP US, NEW YORK, vol. 38, no. 5, 1 May 2020 (2020-05-01), pages 586-599, XP037113520, ISSN: 1087-0156, DOI: 10.1038/S41587-020-0472-9 [retrieved on 2020-05-11]

## Description

### BACKGROUND

The invention relates to the technology of spatial sequencing. The aim is to determine the distribution of mRNA in areas of a tissue or in individual cells within the tissue.

Spatial sequencing is a collective term for methods that allow direct sequencing of the mRNA content of a cell in relation to its tissue context. These methods can on the one hand serve to analyze mRNA expression profiles of cells in a kind of highly multiplexed fluorescence *in situ* hybridization (FISH) assay.

Spatial sequencing in general is disclosed for example in WO2011127099A1 or by Merrit et al, Nature Biotechnology, Vol 38, 2000, 586-599.

On the other hand, *in situ* sequencing can also enable the read-out of mRNA sequence information, using specific mRNA-binding probes, which can take up a copy of predefined portion of specific mRNA or cDNA sequence ("Gap-fill padlock probes", Ke et al., Nature Methods 2013, doi:10.1038/nmeth.2563). All *in situ* sequencing methods require a signal amplification step, which is in most cases performed by circularization of mRNA- or cDNA-binding probes and subsequent rolling circle amplification (RCA), creating a DNA molecule containing multiple copies of the probe sequence, the so called Nanoballs, Rolonies or Rolling circle amplification products (RCPs). As these are large molecules with size in nm or µm scale, the number of rolonies that can be formed within one cell is strictly limited by the size of this cell.

Sequencing involving such padlock-shaped oligonucleotides is disclosed in WO2019038372A1.

Furthermore, if the density of rolonies within cells is too high, discrimination of single mRNA signals during the optical detection step of the sequencing procedure is strongly impaired. As this is a major drawback of the technology, various techniques have been developed to circumvent this, e.g. design of smaller rolonies or generation and clearing of tissue-hydrogel complexes (Asp et al., BioEssays 2020, DOI: 10.1002/bies.201900221). However, these methods do still not fully evade the inherent spatial limitations of *in situ* sequencing.

Another approach avoids *in situ* signal amplification: *In situ* capturing relies on the transfer of mRNA molecules from tissue onto a surface coated with spots of barcoded primers, allowing backtracking of the *ex situ* gained sequence information to the specific tissue region the sequenced mRNA was extracted from. Nevertheless, this method is also limited, as RNA capture efficiency is restricted and resolution is poor (no single-cell analysis) due to the relatively large size of the barcoded capturing spots (Asp et al., BioEssays 2020, DOI:10.1002/bies.201900221).

### SUMMARY

The present invention is directed to a method which uses optical methods to insert a genetic code into a sequence. This code can be used to retrieve the position at which the coding was carried out. The aim here is that the limitations of existing *in situ* sequencing methods with regard to the number of measurable mRNA sequences *in situ* and also the expression dynamics are largely overcome. Optical coding can have a resolution in the range of one µm and a variability of the code that is sufficient for each cell to receive its own code in tissue sections of typical size.

Additionally, optical coding of specific mRNA-binding probes and reverse transcription-mediated "copying" of enclosed mRNAs region into these probes can be performed. The method can thus be used not only to profile the distribution of mRNA (single cell expression profiling) but also to determine the sequence information of certain mRNA sections (e.g., single nucleotide polymorphisms, SNPs; single cell sequencing).

The basic principle of the method as disclosed herein is based on the binding of padlock probes to the mRNA present in the cells. These will subsequently be referred to as MICTAG probes (Mismatch Cell Tagging, or Mismatch Code Tagging).

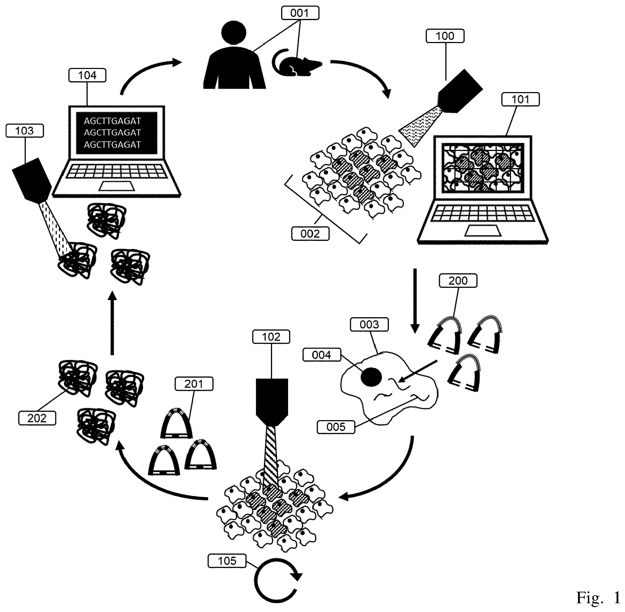

The proposed MICTAG workflow is depicted in Fig. 1: Sample Preparation, tissue staining and imaging (Transmission, Fluorescence) is followed by segmentation or cluster analysis and calculation of masks for the structured illumination. MICTAG Probes bind to specific mRNA molecules within cells, allowing reverse transcriptase-mediated gap-filling (copying of mRNA sequence into MICTAG Probe) and cyclic incorporation of MICTAG Code, rolling circle amplification of MICTAG Probe, sequencing of MICTAG Code. The sequence obtained by gap-filling, as well as the MICTAG Code are annotated to images. Bioinformatics analysis and relation of the results to initial sample source complete the workflow.

Providing of the MICTAG Probes to the mRNA is an essential part of the invention and can be achieved by two general variants.

First object of the invention is a method to obtain the spatial location and sequence information of at least a part of a RNA or cDNA strand (006) in a sample comprising the steps
a. hybridizing a first detection probe oligonucleotide (204) comprising 50 - 1000 nucleotides with its 3' or 5' end to the complementary part of the at least one RNA or cDNA strand, wherein the detection probe oligonucleotide is partially hybridized to a bridge oligonucleotide (205) comprising 5 - 100 nucleotides wherein a gap region (206) capable of binding oligonucleotides is created
b. filling the gap region (206) in part with 1 to 16 barcode oligonucleotides comprising 4 - 20 nucleotides, wherein the barcode oligonucleotides determine the spatial information of the RNA or cDNA strand in the sample
c. partially hybridizing a second detection probe oligonucleotide (204') comprising 50 - 1000 nucleotides with its 3' or 5' end to the complementary part of the same RNA or cDNA strand and with the respective other end to the bridge oligonucleotide (205) to create a circular template
d. multiplying the circular template by a polymerase capable of rolling circle amplification into rolonies comprising a plurality of concatemers
e. determining the sequence of nucleotides of the rolonies

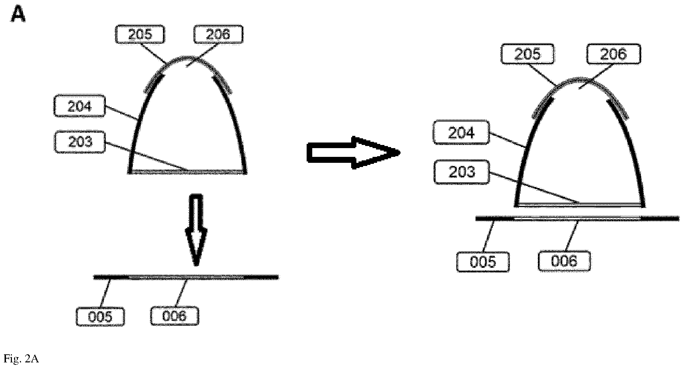

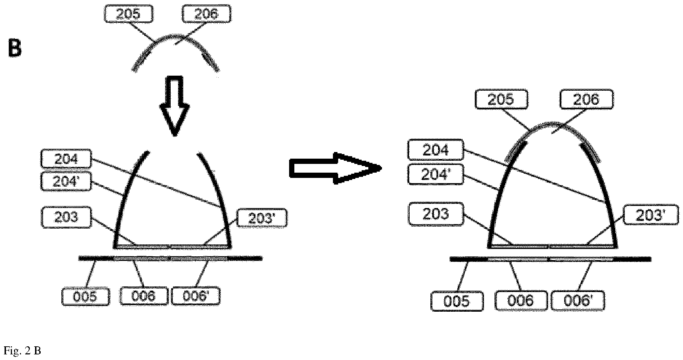

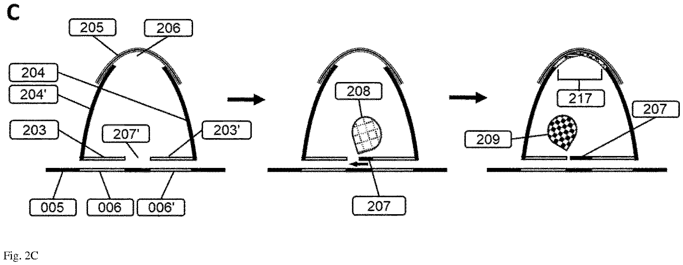

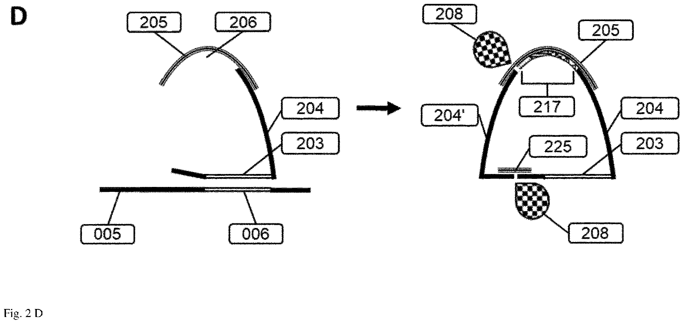

The MICTAG probe may consist of two probes (204, 204') each containing one of the aforementioned specific mRNA binding sites, connected by a partially complementary bound 'bridge' (bridge oligonucleotide (205) in Fig. 2). The sequence of this bridge primer can be the same for all MICTAG probes. Affinity and binding efficiency of the bridge oligonucleotide (205) to its binding regions on the probe can be increased by incorporation of locked nucleic acids (LNA) or peptide nucleic acids (PNA) into the bridge oligomer sequence.

Second object of the invention is a method to obtain the spatial location and sequence information of at least a part of a RNA or cDNA strand ((006)) in a sample comprising the steps
f. hybridizing the 3' and 5' ends of a detection probe oligonucleotide to the complementary parts of the at least one RNA or cDNA strand, wherein the detection probe oligonucleotide comprises a first oligonucleotide (204) and a second oligonucleotide (204'), each comprising 50 - 1000 nucleotides, which are connected by a partially hybridized bridge oligonucleotide (205) comprising 5 - 100 nucleotides wherein a bridge gap region (206) between the first oligonucleotide (204) and second oligonucleotide (204') is created
g. filling the bridge gap region (206) with 1 to 16 barcode oligonucleotides comprising 4 - 20 nucleotides to create a circular template, wherein the barcode oligonucleotides determine the spatial information of the RNA or cDNA in the sample
h. multiplying the circular template by a polymerase capable of rolling circle amplification into rolonies comprising a plurality of concatemers
i. determining the sequence of nucleotides of the rolonies

### BRIEF DESCRIPTION OF THE DRAWINGS

Fig. 1: MICTAG workflow. Sample Preparation, tissue staining and imaging
Fig. 2 : Variants of padlock creation and MICTAG Probe gap-filling
Fig. 1: MICTAG Coding

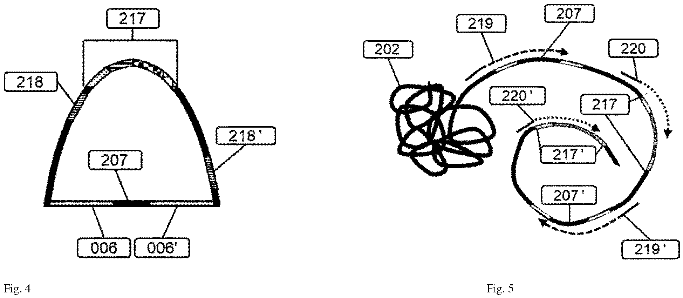

Fig. 4: MICTAG Probe after gap-filling, coding and bridge primer removal.
Fig. 5: Sequencing of MICTAG Rolony.

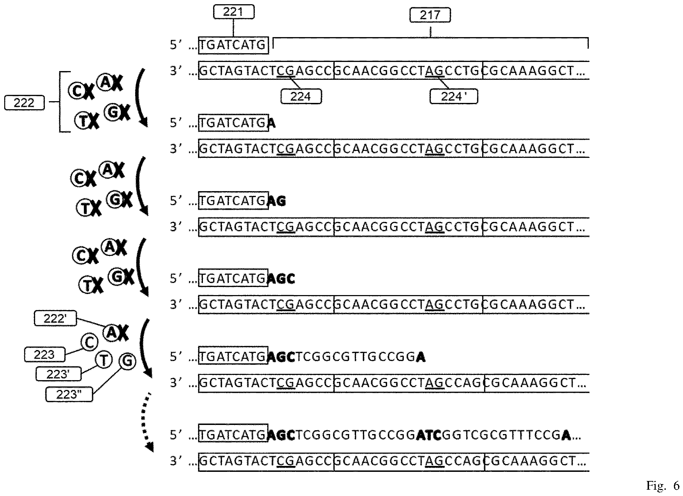

Fig. 6: Accelerated sequencing procedure for MICTAG Codes.

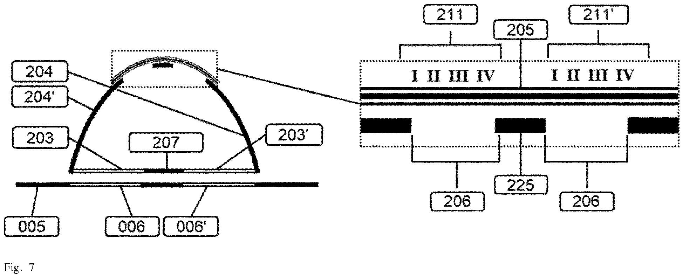

Fig. 7: Parallel coding variant of a MICTAG Probe.

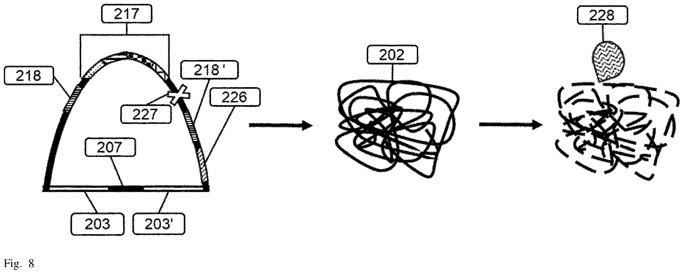

Fig. 8: Incorporation of an unique molecular identifier (UMI) into the MICTAG Probe sequence

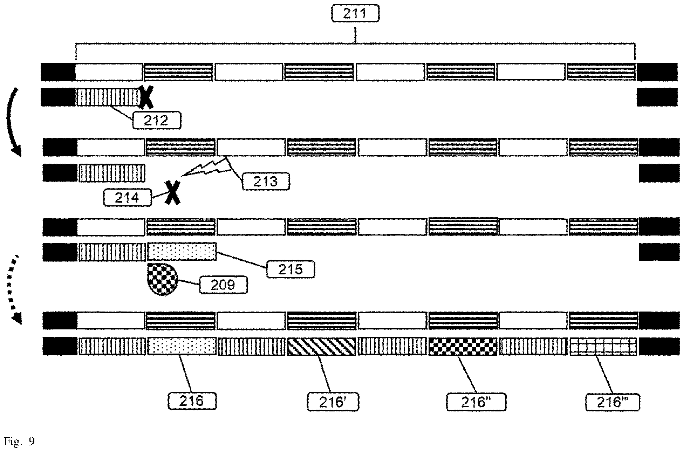

Fig. 9: Primer-controlled MICTAG Coding

### DETAILED DESCRIPTION

In the following, the detection probe oligonucleotide is referred to as "MICTAG Probe", the bridge oligonucleotide (205) contains a plurality of "MICTAG Digits" or shorter as "Digit" and the gap region (206) is filled in with barcode oligonucleotides referred to as "MICTAG Snips" or shorter as "Snip".

The variants to create the detection probe oligonucleotide are shown in Fig. 2 A -D.

In Fig 2 A, the padlock is shown with detection probe oligonucleotide (204) having binding region (203) which binds to the complementary parts (006) of the at least one RNA or cDNA strand (005). The detection probe oligonucleotide is provided with partially hybridized bridge oligonucleotide (205) creating gap region (206) capable of binding oligonucleotides.

In Fig 2 B, the general concept and shape of the padlock is shown with first (204) and second (204') detection probe oligonucleotides have binding regions (203) and (203') which bind to the complementary parts (006) and (006') of the at least one RNA or cDNA strand (005). The final detection probe oligonucleotide is generated by partially hybridizing first (204) and second (204') detection probe oligonucleotides to a bridge oligonucleotide (205) wherein a gap region (206) capable of binding oligonucleotides is created.

In a first variant of the embodiment, shown in Fig. 2B, the detection probe oligonucleotide is hybridized to the at least one RNA or cDNA strand by hybridizing a first detection probe oligonucleotide (204) and a second detection probe oligonucleotide (204), each comprising 50 - 1000 nucleotides with the respective 3' and 5' ends to the complementary part of the at least one RNA or cDNA strand and subsequently connecting the first (204) and second oligonucleotide (204') by partially hybridizing to the bridge oligonucleotide (205).

In a second variant of the embodiment shown in Fig. 2B, the detection probe oligonucleotide is hybridized to the at least one RNA or cDNA strand by ligating a first oligonucleotide (204) to the second oligonucleotide (204') (at parts 203 and 203'), then hybridizing the resulting oligonucleotide to the to the complementary part of the at least one RNA or cDNA strand and subsequently connecting the unbounded ends of the resulting oligonucleotide by partially hybridizing to the bridge oligonucleotide (205).

In the variant shown in Fig. 2 A, the detection probe oligonucleotide and/or the parts of the first and/or second oligonucleotides hybridized to the at least one RNA or cDNA are used to obtain a second target sequence.

Different than to the embodiment shown in Fig. 2A, the embodiment shown in Fig. 2B is provided with a gap (207') between the first oligonucleotide (204) and the second oligonucleotide (204') as hybridized to the mRNA (005).

This variant is shown in Fig. 2C, wherein the detection probe oligonucleotide is hybridized to the complementary parts of the at least one RNA or cDNA strand, thereby creating a gap (207') of 1 to 150 nucleotides between the first oligonucleotide (204) and the second oligonucleotide (204') of the detection probe oligonucleotide.

The gap (207') may then be filled with reverse transcriptase (208) with nucleotides complementary to the adjacent part of the at least one RNA or cDNA strand to obtain a first target sequence (207). The final ligation of the first oligonucleotide (204) and the second oligonucleotide (204') is then performed with DNA ligase (209).

In Fig 2 D, the process of the invention according the its first object is shown. Here, first detection probe oligonucleotide (204) is hybridized with its 3' and/or 5' end (203) to the complementary part ((006)) of the at least one RNA or cDNA strand (005). The first detection probe oligonucleotide (204) may already be provided with and hybridized together with bridge oligonucleotide (205) to the mRNA stand (005).

In alternative, the first detection probe oligonucleotide (204) is first hybridized to the complementary part ((006)) of the at least one RNA or cDNA strand (005) and then partially hybridized to a bridge oligonucleotide (205).

In both cases, a gap region (206) which is capable of binding oligonucleotides is created.

In other words, Fig. 2 (A) shows a MICTAG probe with one mRNA binding site, (B) shows a MICTAG probe with two mRNA binding sites, hybridized to two directly neighboring mRNA regions, and (C) shows a MICTAG probe gap-filling: After the MICTAG probe has bound to two not directly neighboring mRNA regions of a specific mRNA species, cDNA synthesis for gap-filling is performed by reverse transcriptase. A DNA ligase connects the strands.

This kind of single-stranded DNA probe contains two specific mRNA binding regions that hybridize with two neighboring regions or two nearby, but not directly neighboring regions of the mRNA. The gap created when probe binding occurs at two nearby, but not directly neighboring regions, can then be "filled up" ('gap-filling') with a sequence complementary to the enclosed mRNA via reverse transcriptase.

Subsequently, the probe is circularized by a DNA ligase, allowing later rolling circle amplification (209 in Fig. 2).

In the following process steps, the gap region (206) is filled step by step, inserting a piece of the complementary sequence (MICTAG Snip) in each step to the complementary MICTAG Digit and fixing it with light. In contrast to the cyclic chemistry used e.g. in a Sequencing by Synthesis process, in one embodiment no single deoxynucleotides are inserted, but small oligonucleotides (MICTAG Snip).

Accordingly, the bridge gap region (206) may at least in part filled by hybridizing barcode oligonucleotides comprising the same or different photocleavable blocking groups to complementary parts of the bridge oligonucleotide (205) by removing the photocleavable blocking group with light after hybridizing.

### Mismatching

The MICTAG Snips (barcode oligonucleotides) can have one or more mismatches with the corresponding MICTAC Digit, depending on their length, but should have a sufficient sequence length to still bind specifically to the (almost) complementary parts of the bridge oligonucleotide.

The term "mismatch" refers to barcode oligonucleotides which have at least one non-complementary nucleotide.

A MICTAG Snip can contain one or two mismatches with a preferred ratio MICTAG Snip : mismatch = 10: 1 bp. In addition to the mismatch versions, MICTAG Snips might also contain the matching code, serving as another variant.

To assure specific hybridization with their associated MICTAG Digits, Snips need to be designed in such a way that they cannot bind other Digits. As an alternative to mismatches in Snips, bridge primers can also be designed in such a way that they contain universal bases at the complementary positions to Snip mismatches.

Annealing of these MICTAG Snips is performed within a cyclic process, which is triggered by a structured illumination of the tissue sample by treating it with another spatially structured pattern of light in each cycle. "Structured illumination" and "spatially structured pattern of light" refer to illuminating only a part or selected areas of the sample. For example, Fig. 1 shows a tissue section 002 (optionally stained) obtained from tissue donor 001 which is subjected to imaging 100, allowing segmentation or cluster analysis, i.e. the selection of the parts of the sample to be further investigated by the method of the invention. Such segmentation/clustering/selection enables calculation of masks for the structured illumination and/or for spatially structured pattern of light.

Further downstream in the method of the invention, the information obtained for structured illumination and/or for spatially structured pattern of light is utilized during photo-treatment for MICTAG Code generation (102) which enables circularization of the padlock probes 201 and finally, after RCA, formation of rolonies 202. With the aid of the structured illumination, only the selected areas/cells of the sample 202 are subjected to Next Generation Sequencing of rolonies (100) and sequence analysis (101).

The kinetics of binding of the MICTAG Snips is adjusted so that if a photocleavable group is not removed, the Snip can be detached and washed off, e.g. by increasing the temperature. If the exposure has been made, the MICTAG Snips, hybridized with their corresponding MICTAG Digit within the bridge oligonucleotide, are fixed within the probe, as a photocleavable group is detached by patterned illumination, allowing ligase reaction at this site. Finally, the individual combination of MICTAG Snips determines the MICTAG Code that encodes the location of the MICTAG probe within a tissue section or even within a cell.

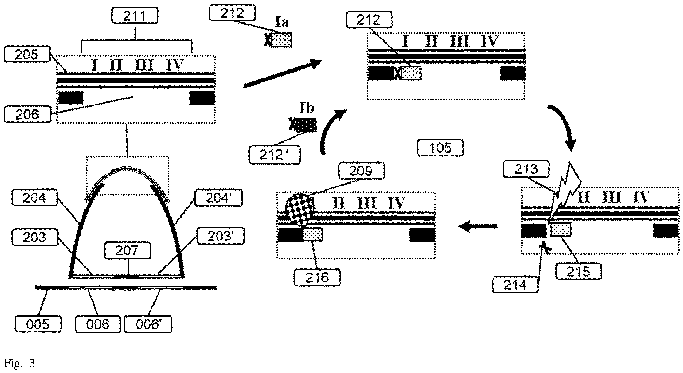

The process of MICTAG coding is shown in Fig. 3: One of four specific gap-filling oligos is added to the bound and gap-filled MICTAG probe, having either none, one or two base mismatches. With this scheme, 16 different mismatch probes can be designed for each MICTAG digit, binding one of four MICTAG Digit binding regions (I-IV). Illumination of single cells induces photo-cleavage and allows incorporation of MICTAG Snips by a DNA ligase.

Starting from the MICTAG probe where gap filling with reverse transcriptase of the target strand has already been done, the MICTAG Snips are built in in a cyclic process until the code, which consists of several Snips, is written. In the example shown in Fig. 3, the variability of the Snips is 16, which can be achieved by 2 mismatches and the 4 binding regions within the bridge (MICTAG Digits). So, it requires 4 × 16 cycles to write a code that can have 65536 different values.

After the coding has been completed, all MICTAG probes i.e. the circular templates are isolated i.e. removed from the tissue sample. The bridge oligonucleotides may be detached from circular templates, e.g., by heat-induced double strand melting. In an alternative variant, the bridge oligonucleotides are provided with a primer sequence to induce rolling circle amplification and thus can remain on the circular templates / MICTAG probes.

Fig. 4 shows the isolated MICTAG Probe that carries the MICTAG Snips (217), providing information from which tissue structure, cell, or subcellular region the mRNA strand originates. In addition, due to cDNA synthesis by gap-filling (207) between the mRNA binding sites of the MICTAG probe ((006), (006')), information about eventual mutations or SNPs (single nucleotide polymorphisms) of the targeted mRNA is included (Fig. 4, 208).

In principle, the method is not limited in terms of the number of mRNA types examined simultaneously, nor is it limited in terms of individual mRNA expression levels, so that high-level expressers as housekeeping genes will not impair the process. The number of cells examined individually at the same time is limited by the chosen code length. The limitation is only in the capacity and throughput of the sequencer.

### Sequencing

One step in the method of the invention is directed to determine the sequence of nucleotides of the rolonies i.e. the information encoded on the MICTAG probes is read out by sequencing. One method for sequencing can be sequencing be synthesis (SBS). For increasing readout signals, amplification of the MICTAG probe sequences can be performed. One method for clonal amplification can be rolling circle amplification (RCA) of the isolated MICTAG probes, which is performed before starting the sequencing process on the rolonies.

Fig. 5 shows the sequencing of MICTAG rolony. Two sequencing primers guide the consecutive sequencing of gap-fill cDNA sequence (218, 218') and MICTAG Code (219, 219').

In a variant of the invention, the sequence of the MICTAG code may be read separately from the sequence of the target gene (gap-fill sequence). This can be realized by splitting up the sequencing procedure into two runs with two different sequencing primers.

### Acceleration of MICTAG-code sequencing

The sequencing of the MICTAG Snips can be accelerated if the matching code before and after the mismatching region consists of only a sequence of 3 of the four bases (e.g. G,T,C). In the following sequence M is a placeholder for one of the bases G, A, C and X is a placeholder for the mismatching code:
MMMMMTXXMMMMM (MICTAG Snip with M ={G, A, C} und X ={A, G, T, C}

In the context of cyclic chemistry applied for sequencing by synthesis, incorporated nucleotides are usually modified with a base-specific fluorophore and a terminator (indicated here by A*, G*, C*, T*), which blocks further polymerase-mediated strand-elongation until the position is read. When reading the MICTAG Snip in the variant given above, a mixture of nucleotides T, G, C without dye and terminator and A* can be applied, which are then incorporated until T occurs, always preceding the mismatch, prevents further elongation by incorporating an A*.

The mismatch portion of the sequence can then be detected by using a mixture of nucleotides modified with terminator and fluorescent dye. Then, the T, G, C, A* mixture is added again and strand elongation proceeds until the next T in front of the next mismatch position in the following MICTAG Snip.

This can essentially reduce the number of cycles required to sequence the mismatch positions in the MICTAG Code. Using this strategy, dephasing of the cyclic incorporation is avoided and corrected. The first MICTAG Snip sequence to be read can also be designed in such a way that the sequencing primer binds just before the mismatch sequence, thus eliminating the need for initial padding with the T, G, C, A*.

This variant of the invention is shown in Fig. 6 where for example two consecutive mismatches are present in the MICTAG Snips. In Fig. 6, an accelerated sequencing procedure for MICTAG Codes is shown. Guided by a specific sequencing primer, fluorescently labeled nucleotides with terminator (T*, C*, G*, A*) are added in 3 cycles to enable mismatch detection in a sequencing by synthesis approach. In the following step, non-labeled and non-terminated nucleotides (T, C, G) and fluorescently labeled A with terminator (A*) are added and the strand is filled up by DNA polymerase. Binding of A* stops this step, as it indicates that the next mismatch position has been reached. Again, fluorescently labeled nucleotides with terminator (T*, C*, G*, A*) are added in 3 cycles to enable mismatch detection in a sequencing by synthesis approach. This approach is repeated until the whole MICTAG Code is sequenced and requires specifically designed MICTAG Snips that do not contain Thymidine within their sequence, with exception of the mismatch regions.

The variant utilizes by way of example the following sequences :
SEQ1: 3` end of sequencing primer 221: 5`-...TGATCATG-3' (less than 10 nucleotides, no sequence listing)
SEQ2: First MICTAG Snip (with mismatch nucleotides 224): 3'-GCTAGTACTCGAGCC-5'
SEQ3: Second MICTAG Snip (with mismatch nucleotides 224'): 3'-GCAACGGCCTAGCCTG-5'
SEQ4: 3' end of third MICTAG Snip: 3'-CGCAAAGGCT.. . -5'
SEQ5: 5' end of complementary sequence generated in sequencing by synthesis procedure (with labeled nucleotides 222): 5'-... TGATCATGAGCTCGGCGTTGCCGGATCGGTCGCGTTTCCGA... -3'

### Different MICTAG Codes

The MICTAG code can be generated by different MICTAG Snip combinations. Here, the variability N of the Snips represents the number of different Snips. Preferable, the number of different Snips is N = 2, 4 or 16. The number of MICTAG Digits, M (number of binding regions), might be in the range between 1 and 32. The total variability of the MICTAG Code is then NM. The number of cycles needed to write the code is N × M.

### Acceleration of the code generation

After the incorporation of the (N-1) types of MICTAG Snips at each of the MICTAG Digits (binding sites) the last type of the MICTAG Snips can be incorporated without the application of light, since it is related to the complementary entity of the already illuminated areas within the first N-1 steps. This can be utilized by providing a Snip having no photocleavable group. This non-photocleavable Snip can then be provided together with the first (photocleavable) MICTAG Snip for the following MICTAG Digit. Counting this as a single cycle reduces the required cycles to N × M - M + 1 = (N - 1) × M +1. The last cycle would than complete the MICTAG code without the application of light. This method is especially useful in the case N = 2 where N × M - M + 1 = M + 1.

The following table shows the examples of the variants of the method of the invention and their effect on the number of required cycles for MICTAG coding.

| #MICTAG Snips N | #MICTAG Digits M | MICTAG Code variability N^{M} | Number of cycles needed for code generation N × M (accelerated: (N - 1) × M + 1) |
|---|---|---|---|
| 2 | 8 | 256 | 16(9) |
| 2 | 16 | 65565 32 (17) | 32(17) |
| 4 | 8 | 65565 | 32 (25) |
| 16 | 4 | 65565 | 64 (61) |

### Error handling

When the MICTAG Code is written, errors may occur when the MICTAG Snips are incorporated. If one of the MICTAG Snips is not inserted, the probe will not be circularized, no RCA can take place and subsequently no sequencing information will be generated.

If an incorrect MICTAG Snip is inserted, the code will not refer to the correct cell. This restriction is only valid if the acceleration of the code generation outlined in the preceding section is not applied.

In other words, if a MICTAG Snip is not inserted, no padlock will be created which will in term not by subjected to sequencing. Insofar, the method of the invention has a build-in error handling.

In addition, it can make sense to include other error correction methods. For example, a second identical code can be inserted at the same time into the bridge at a different position, so that one can validate the codes internally. It is also possible to increase the code length and to add error correction by redundancy.

In this variant, the bridge oligonucleotide contains at least two identical binding regions for the same MICTAG Snip, which are accessible at the same time to assure specific binding reactions. Again, if one MICTAG Snip is not inserted correctly, the respective padlock is not created and will not be subjected to sequencing.

In this respect, Fig. 7 shows a MICTAG Probe with two identical regions, which are written identically in parallel. It is then possible to assign only those sequences to a location where both codes are identical. This prevents that especially in samples with very different mRNA expression profiles among different sample regions, these contrasts are leveled out by incorrect codes.

The readout of the genetic code suffers from readout errors. These errors can occur in the MICTAG code as well as in the mRNA sequence. Therefore, it may be helpful to enzymatically cut the strand produced by RCA and generate multiple strands from each strand again by RCA for sequencing. It is then helpful to include a unique molecular identifier (UMI) in the MICTAG probe so that error correction can be performed on both the MICTAG code and the mRNA sequence. This variant is shown in Fig. 8 with the incorporation of unique molecular identifier (UMI) 226 into the MICTAG probe sequence. The UMI can be used to identify the original MICTAG probe after enzymatic digestion of the rolony.

### Alternative coding approaches

In addition to the suggested MICTAG procedure, alternative methods can be used to write a code into a MICTAG Probe *in situ.* One variant of the so far described MICTAG procedure is depicted in Fig. 9 Here, ligase-mediated MICTAG Snip incorporation can be controlled by additional primers, which need to be incorporated beforehand. As the MICTAG Snips, theses primers carry photolabile group that are removed by illumination with specific wavelength light, allowing their subsequent incorporation into the strand by DNA ligase. Release of a fluorophore optionally coupled to photolabile groups can be detected and may serve as internal control.

Instead of the additional primers mentioned in Fig. 9 single nucleotides, carrying a photocleavable group, can be used. In contrast to the primers and MICTAG Snips, which hybridize with complementary strands of the bridge probe and are integrated into to the probe by DNA ligase, the nucleotide needs to be incorporated by a DNA polymerase.

Fig. 9 shows a variant of the invention with primer-controlled MICTAG coding. Here, primer, modified with a photolabile group is hybridized to the single-stranded region next to the first MICTAG Digit; Light induces the cleavage of the photolabile group coupled to the primer; The primer is incorporated into the strand by a DNA ligase, then the first Snip is added and hybridizes with the first Digit. This step is repeated until all different Snips at the Digit I location are incorporated; Now the first is repeated to prepare the incorporation of the different types of Snip II at the Digit II; After multiple cycles, the MICTAG Code is complete and the MICTAG probe is finally ligated.

### Glossar to the drawings

### Organisms, tissue and tissue content

- 001: Tissue donor
- 002: Stained tissue section
- 003: Cell
- 004: Cell nucleus
- 005: mRNA
- 006: MICTAG Probe binding sites on mRNA

### Technical procedures

- 100: Imaging
- 101: Segmentation or cluster analysis, calculation of masks for the structured illumination
- 102: Photo-treatment for MICTAG Code generation
- 103: Next generation sequencing of rolonies
- 104: Sequence analysis
- 105: Cyclic barcoding

### Reagents and generated products

- 200: MICTAG Probe before cDNA gap-filling and individual coding
- 201: MICTAG Probe after cDNA gap-filling and individual coding
- 202: Rolony or Rolling circle amplification product (RCP)
- 203: Specific mRNA binding site
- 204: MICTAG probe backbone
- 205: Bridge oligonucleotide hybridized to MICTAG Probe backbone ends
- 206: Gap created by bridge oligonucleotide hybridization to MICTAG probe backbone
- 207: cDNA (or gap for cDNA, 207')
- 208: Reverse transcriptase
- 209: DNA ligase
- 210: MICTAG Probe after gap-filling
- 211: Bridge primer with MICTAG Digits (I-IV)
- 212: Oligonucleotide or MICTAG Snip with photolabile protective group
- 213: Focused light cleaving photolabile protective group off the MICTAG Snip
- 214: Cleaved-off photolabile protective group
- 215: MICTAG Snip without photolabile protective group
- 216: MICTAG Snip integrated into MICTAG Probe
- 217: MICTAG Code
- 218: Sequencing primer binding site
- 219: Sequencing procedure mRNA binding sites and cDNA
- 220: Sequencing procedure MICTAG Code
- 221: Sequencing primer
- 222: Nucleotide with (photolabile) protective group
- 223: Nucleotide
- 224: Variable nucleotides in MICTAG Code ("Mismatches")
- 225: Splint oligonucleotide supporting DNA ligase activity
- 226: Unique molecular identifier (UMI)
- 227: Restriction site
- 228: Restriction enzyme

## Claims

1. A method to obtain the spatial location and sequence information of at least a part of a RNA or cDNA strand ((006)) in a sample comprising the steps
a. hybridizing a first detection probe oligonucleotide (204) comprising 50 - 1000 nucleotides with its 3' or 5' end to the complementary part of the at least one RNA or cDNA strand, wherein the detection probe oligonucleotide is partially hybridized to a bridge oligonucleotide (205) comprising 5 - 100 nucleotides wherein a gap region (206) capable of binding oligonucleotides is created
b. filling the gap region (206) in part with 1 to 16 barcode oligonucleotides comprising 4 - 20 nucleotides, wherein the barcode oligonucleotides determine the spatial information of the RNA or cDNA strand in the sample
c. partially hybridizing a second detection probe oligonucleotide (204') comprising 50 - 1000 nucleotides with its 3' or 5' end to the complementary part of the same RNA or cDNA strand and with the respective other end to the bridge oligonucleotide (205) to create a circular template
d. multiplying the circular template by a polymerase capable of rolling circle amplification into rolonies comprising a plurality of concatemers
e. determining the sequence of nucleotides of the rolonies

2. A method to obtain the spatial location and sequence information of at least a part of a RNA or cDNA strand ((006)) in a sample comprising the steps
f. hybridizing the 3' and 5' ends of a detection probe oligonucleotide to the complementary parts of the at least one RNA or cDNA strand, wherein the detection probe oligonucleotide comprises a first oligonucleotide (204) and a second oligonucleotide (204'), each comprising 50 - 1000 nucleotides, which are connected by a partially hybridized bridge oligonucleotide (205) comprising 5 - 100 nucleotides wherein a bridge gap region (206) between the first oligonucleotide (204) and second oligonucleotide (204') is created
g. filling the bridge gap region (206) with 1 to 16 barcode oligonucleotides comprising 4 - 20 nucleotides to create a circular template, wherein the barcode oligonucleotides determine the spatial information of the RNA or cDNA in the sample
h. multiplying the circular template by a polymerase capable of rolling circle amplification into rolonies comprising a plurality of concatemers
i. determining the sequence of nucleotides of the rolonies

3. Method according to claim 2 **characterized in that** the detection probe oligonucleotide is hybridized to the at least one RNA or cDNA strand by hybridizing a first detection probe oligonucleotide (204) and a second detection probe oligonucleotide (204), each comprising 50 - 1000 nucleotides with the respective 3' and 5' ends to the complementary part of the at least one RNA or cDNA strand and subsequently connecting the first (204) and second oligonucleotide (204') by partially hybridizing to the bridge oligonucleotide (205).

4. Method according to claim 2 **characterized in that** the detection probe oligonucleotide is hybridized to the at least one RNA or cDNA strand by ligating a first oligonucleotide (204) to the second oligonucleotide (204'), then hybridizing the resulting oligonucleotide to the to the complementary part of the at least one RNA or cDNA strand and subsequently connecting the unbounded ends of the resulting oligonucleotide by partially hybridizing to the bridge oligonucleotide (205).

5. Method according to any of the claims 1-4 **characterized in that** the detection probe oligonucleotide is hybridized to the complementary parts of the at least one RNA or cDNA strand, thereby creating a gap (207') of 1 to 150 nucleotides between the first oligonucleotide (204) and the second oligonucleotide (204') of the detection probe oligonucleotide.

6. Method according to claim 5 **characterized in that** the gap (207') is filled with nucleotides complementary to the adjacent part of the at least one RNA or cDNA strand to obtain a first target sequence (207).

7. Method according to any of the claims 1-6 **characterized in that** the parts of the first and/or second oligonucleotides hybridized to the at least one RNA or cDNA are used to obtain a second target sequence.

8. Method according to claim 6 or 7 **characterized in that** the spatial information of the circular template in the sample is linked to the first and/or second target sequence.

9. Method according to any of the claims 1 - 8 **characterized in that** the bridge gap region (206) is at least in part filled by hybridizing barcode oligonucleotides comprising the same or different photocleavable blocking groups to complementary parts of the bridge oligonucleotide (205) by removing the photocleavable blocking group with light after hybridizing.

10. Method according to any of the claims 1 to 9 **characterized in that** the circular template is multiplied selectively by providing primer oligonucleotides complementary to one of the barcode oligonucleotides as priming site for a rolling circle amplification polymerase.

11. Method according to any of the claims 1 to 10 **characterized in that** the sample is fixed and permeabilized on a surface.

12. Method according to any of the claims 1 to 11 **characterized in that** the sample is provided as tissue and the single strand circular template is isolated from the sample and replicated ex situ by rolling circle amplification.

13. Method according to any of the claims 1 to 11 **characterized in that** the sample is provided as tissue and the single strand circular template is replicated on the tissue by rolling circle amplification.

## Patentansprüche

1. Verfahren zur Gewinnung der räumlichen Lage und der Sequenzinformation von mindestens einem Teil eines RNA- oder cDNA-Strangs ((006)) in einer Probe, umfassend die Schritte:
a. Hybridisieren eines ersten Nachweissonden-Oligonukleotids (204), das 50 - 1.000 Nukleotide umfasst, mit seinem 3'- oder 5'-Ende an den komplementären Teil des mindestens einen RNA- oder cDNA-Strangs, wobei das Nachweissonden-Oligonukleotid teilweise an ein Brücken-Oligonukleotid (205), das 5 - 100 Nukleotide umfasst, hybridisiert wird, wobei eine Lückenregion (206), die Oligonukleotide binden kann, erzeugt wird,
b. teilweises Auffüllen der Lückenregion (206) mit 1 bis 16 Barcode-Oligonukleotiden, die 4 - 20 Nukleotide umfassen, wobei die Barcode-Oligonukleotide die räumliche Information des RNA- oder cDNA-Strangs in der Probe bestimmen,
c. teilweises Hybridisieren eines zweiten Oligonukleotids (204'), das 50 - 1.000 Nukleotide umfasst, mit seinem 3'- oder 5'-Ende an den komplementären Teil desselben RNA- oder cDNA-Strangs und mit dem jeweils anderen Ende an das Brücken-Oligonukleotid (205), um eine zirkuläre Matrize zu erzeugen,
d. Vervielfältigen der zirkulären Matrize durch eine Polymerase, die zur Rolling-Circle-Amplifikation fähig ist, zu Rolonies, die eine Vielzahl von Concatemeren umfassen,
e. Bestimmen der Nukleotidsequenz der Rolonies.

2. Verfahren zur Gewinnung der räumlichen Lage und der Sequenzinformation von mindestens einem Teil eines RNA- oder cDNA-Strangs ((006)) in einer Probe, umfassend die Schritte:
f. Hybridisieren der 3'- und 5'-Enden eines Nachweissonden-Oligonukleotids an die komplementären Teile des mindestens einen RNA- oder cDNA-Strangs, wobei das Nachweissonden-Oligonukleotid ein erstes Oligonukleotid (204) und ein zweites Oligonukleotid (204') umfasst, die jeweils 50 - 1.000 Nukleotide umfassen, die durch ein teilweise hybridisiertes Brücken-Oligonukleotid (205) verbunden sind, das 5 - 100 Nukleotide umfasst, wobei eine Brücken-Lückenregion (206) zwischen dem ersten Oligonukleotid (204) und dem zweiten Oligonukleotid (204') erzeugt wird,
g. Auffüllen der Brücken-Lückenregion (206) mit 1 bis 16 Barcode-Oligonukleotiden, die 4 bis 20 Nukleotide umfassen, um eine zirkuläre Matrize zu erzeugen, wobei die Barcode-Oligonukleotide die räumliche Information der RNA oder cDNA in der Probe bestimmen,
h. Vervielfältigen der zirkulären Matrize durch eine Polymerase, die zur Rolling-Circle-Amplifikation fähig ist, zu Rolonies, die eine Vielzahl von Concatemeren umfassen,
i. Bestimmen der Nukleotidsequenz der Rolonies.

3. Verfahren nach Anspruch 2, **dadurch gekennzeichnet, dass** das Nachweissonden-Oligonukleotid an den mindestens einen RNA- oder cDNA-Strang hybridisiert wird, indem ein erstes Nachweissonden-Oligonukleotid (204) und ein zweites Nachweissonden-Oligonukleotid (204) hybridisiert werden, wobei jedes Oligonukleotid 50 - 1.000 Nukleotide mit den jeweiligen 3'- und 5'-Enden an den komplementären Teil des mindestens einen RNA- oder cDNA-Strangs umfasst, und anschließend das erste (204) und das zweite Oligonukleotid (204') durch teilweise Hybridisierung mit dem Brücken-Oligonukleotid (205) verbunden werden.

4. Verfahren nach Anspruch 2, **dadurch gekennzeichnet, dass** das Nachweissonden-Oligonukleotid an den mindestens einen RNA- oder cDNA-Strang hybridisiert wird, indem ein erstes Oligonukleotid (204) an das zweite Oligonukleotid (204') ligiert wird, anschließend Hybridisieren des resultierenden Oligonukleotids an den komplementären Teil des mindestens einen RNA- oder cDNA-Strangs und anschließendes Verbinden der nicht gebundenen Enden des resultierenden Oligonukleotids durch teilweises Hybridisieren an das Brücken-Oligonukleotid (205).

5. Verfahren nach einem der Ansprüche 1 bis 4, **dadurch gekennzeichnet, dass** das Nachweissonden-Oligonukleotid an die komplementären Teile des mindestens einen RNA- oder cDNA-Strangs hybridisiert wird, wodurch eine Lücke (207') von 1 bis 150 Nukleotiden zwischen dem ersten Oligonukleotid (204) und dem zweiten Oligonukleotid (204') des Nachweissonden-Oligonukleotids erzeugt wird.

6. Verfahren nach Anspruch 5, **dadurch gekennzeichnet, dass** die Lücke (207') mit Nukleotiden gefüllt wird, die zu dem benachbarten Teil des mindestens einen RNA- oder cDNA-Strangs komplementär sind, um eine erste Zielsequenz (207) zu erhalten.

7. Verfahren nach einem der Ansprüche 1 bis 6, **dadurch gekennzeichnet, dass** die mit der mindestens einen RNA oder cDNA hybridisierten Teile der ersten und/oder zweiten Oligonukleotide zur Gewinnung einer zweiten Zielsequenz verwendet werden.

8. Verfahren nach Anspruch 6 oder 7, **dadurch gekennzeichnet, dass** die räumliche Information der zirkulären Matrize in der Probe mit der ersten und/oder zweiten Zielsequenz verknüpft ist.

9. Verfahren nach einem der Ansprüche 1 bis 8, **dadurch gekennzeichnet, dass** die Brücken-Lückenregion (206) zumindest teilweise durch Hybridisierung von Barcode-Oligonukleotiden, die die gleichen oder unterschiedliche photospaltbare Blockierungsgruppen umfassen, an komplementäre Teile des Brücken-Oligonukleotids (205) gefüllt wird, indem die photospaltbare Blockierungsgruppe nach der Hybridisierung mit Licht entfernt wird.

10. Verfahren nach einem der Ansprüche 1 bis 9, **dadurch gekennzeichnet, dass** die zirkuläre Matrize selektiv vervielfältigt wird, indem Primer-Oligonukleotide, die zu einem der Barcode-Oligonukleotide komplementär sind, als Primingstelle für eine Rolling-Circle-Amplifikationspolymerase bereitgestellt werden.

11. Verfahren nach einem der Ansprüche 1 bis 10, **dadurch gekennzeichnet, dass** die Probe auf einer Oberfläche fixiert und permeabilisiert wird.

12. Verfahren nach einem der Ansprüche 1 bis 11, **dadurch gekennzeichnet, dass** die Probe als Gewebe bereitgestellt wird und die einzelsträngige zirkuläre Matrize aus der Probe isoliert und ex situ durch Rolling-Circle-Amplifikation repliziert wird.

13. Verfahren nach einem der Ansprüche 1 bis 11, **dadurch gekennzeichnet, dass** die Probe als Gewebe bereitgestellt wird und die einzelsträngige zirkuläre Matrize auf dem Gewebe durch Rolling-Circle-Amplifikation repliziert wird.

## Revendications

1. Procédé d'obtention de la localisation spatiale et des informations de séquence d'au moins une partie d'un brin d'ARN ou d'ADNc ((006)) dans un échantillon comprenant les étapes consistant à
a. hybrider un premier oligonucléotide de sonde de détection (204) comprenant 50 à 1 000 nucléotides avec son extrémité 3' ou 5' à la partie complémentaire de l'au moins un brin d'ARN ou d'ADNc, l'oligonucléotide de sonde de détection étant partiellement hybridé à un oligonucléotide de pontage (205) comprenant 5 à 100 nucléotides une région d'espace libre (206) capable de se lier aux oligonucléotides étant créée
b. remplir la région d'espace libre (206) en partie avec 1 à 16 oligonucléotides de code barre comprenant 4 à 20 nucléotides, les oligonucléotides de code barre déterminant les informations spatiales du brin d'ARN ou d'ADNc dans l'échantillon
c. hybrider partiellement un second oligonucléotide de sonde de détection (204') comprenant 50 à 1 000 nucléotides avec son extrémité 3' ou 5' à la partie complémentaire du même brin d'ARN ou d'ADNc et avec l'autre extrémité respective à l'oligonucléotide de pontage (205) pour créer une matrice circulaire
d. multiplier la matrice circulaire par une polymérase capable d'amplification en cercle roulant en rolonies comprenant une pluralité de concatémères
e. déterminer la séquence nucléotidique des rolonies.

2. Procédé d'obtention de la localisation spatiale et des informations de séquence d'au moins une partie d'un brin d'ARN ou d'ADNc ((006)) dans un échantillon comprenant les étapes consistant à
f. hybrider les extrémités 3' et 5' d'un oligonucléotide de sonde de détection aux parties complémentaires de l'au moins un brin d'ARN ou d'ADNc, l'oligonucléotide de sonde de détection comprenant un premier oligonucléotide (204) et un second oligonucléotide (204'), chacun comprenant 50 à 1 000 nucléotides, qui sont connectés par un oligonucléotide de pontage partiellement hybridé (205) comprenant 5 à 100 nucléotides une région d'espace libre de pontage (206) entre le premier oligonucléotide (204) et le second oligonucléotide (204') étant créée
g. remplir la région d'espace libre de pontage (206) avec 1 à 16 oligonucléotides de code barre comprenant 4 à 20 nucléotides pour créer une matrice circulaire, les oligonucléotides de code barre déterminant les informations spatiales de l'ARN ou de l'ADNc dans l'échantillon
h. multiplier la matrice circulaire par une polymérase capable d'amplification en cercle roulant en rolonies comprenant une pluralité de concatémères
i. déterminer la séquence nucléotidique des rolonies.

3. Procédé selon la revendication 2, **caractérisé en ce que** l'oligonucléotide de sonde de détection est hybridé à l'au moins un brin d'ARN ou d'ADNc en hybridant un premier oligonucléotide de sonde de détection (204) et un second nucléotide de sonde de détection (204), chacun comprenant 50 à 1 000 nucléotides avec les extrémités respectives 3' et 5' à la partie complémentaire d'au moins un brin d'ARN ou d'ADNc et ensuite connecter le premier (204) et le second (204') oligonucléotides par hybridation partielle à l'oligonucléotide de pontage (205).

4. Procédé selon la revendication 2, **caractérisé en ce que** l'oligonucléotide de sonde de détection est hybridé à l'au moins un brin d'ARN ou d'ADNc par ligation d'un premier oligonucléotide (204) au second oligonucléotide (204'), puis hybridation de l'oligonucléotide résultant à la partie complémentaire de l'au moins un brin d'ARN ou d'ADNc et ensuite connecter les extrémités non liées de l'oligonucléotide résultant à l'oligonucléotide de pontage (205).

5. Procédé selon l'une quelconque des revendications 1 à 4, **caractérisé en ce que** l'oligonucléotide de sonde de détection est hybridé aux parties complémentaires de l'au moins un brin d'ARN ou d'ADNc, créant ainsi un espace libre (207') de 1 à 150 nucléotides entre le premier oligonucléotide (204) et le second nucléotide (204') de l'oligonucléotide de sonde de détection.

6. Procédé selon la revendication 5, **caractérisé en ce que** l'espace libre (207') est rempli avec des nucléotides complémentaires à la partie adjacente de l'au moins un brin d'ARN ou d'ADNc pour obtenir une première séquence cible (207).

7. Procédé selon l'une quelconque des revendications 1 à 6, **caractérisé en ce que** les parties des premier et/ou second oligonucléotides hybridées à l'au moins un ARN ou ADNc sont utilisées pour obtenir une seconde séquence cible.

8. Procédé selon la revendication 6 ou 7, **caractérisé en ce que** les informations spatiales de la matrice circulaire dans l'échantillon sont liées à la première et/ou la seconde séquences cibles.

9. Procédé selon l'une quelconque des revendications 1 à 8, **caractérisé en ce que** la région d'espace libre de pontage (206) est au moins en partie remplie par les oligonucléotides de code barre d'hybridation comprenant les mêmes ou différents groupes de blocage photoclivables aux parties complémentaires de l'oligonucléotide de pontage (205) en enlevant le groupe de blocage photoclivable avec de la lumière après l'hybridation.

10. Procédé selon l'une quelconque des revendications 1 à 9, **caractérisé en ce que** la matrice circulaire est multipliée sélectivement en fournissant des oligonucléotides d'amorce complémentaires à un des oligonucléotides de code barre comme site d'amorçage pour une polymérase d'amplification à cercle roulant.

11. Procédé selon l'une quelconque des revendications 1 à 10, **caractérisé en ce que** l'échantillon est fixé et perméabilisé sur une surface.

12. Procédé selon l'une quelconque des revendications 1 à 11, **caractérisé en ce que** l'échantillon est fourni comme un tissu et la matrice circulaire simple brin est isolée de l'échantillon et répliquée ex situ par amplification en cercle roulant.

13. Procédé selon l'une quelconque des revendications 1 à 11, **caractérisé en ce que** l'échantillon est fourni comme un tissu et la matrice circulaire simple brin est répliquée sur le tissu par amplification en cercle roulant.
